# EUROPÄISCHE PATENTSCHRIFT

(11) **EP 2 124 628 B1**
(45) Veröffentlichungstag und Bekanntmachung des Hinweises auf die Patenterteilung: **17.04.2013**
(21) Anmeldenummer: 08707794.7
(22) Anmeldetag: 22.02.2008
(51) Int. Cl.: A23L 1/22, A23L 1/236, A23L 1/29, A23G 1/40, A23L 1/30, A23D 7/015, A61K 31/7016, A23C 9/152, A23C 9/156, A23D 7/005

(54) **NIEDRIG-GLYKÄMISCHE MISCHUNGEN**
LOW GLYCEMIC MIXTURES
MÉLANGES À FAIBLE GLYCÉMIE

(30) Priorität: 23.02.2007 DE 102007009029
(43) Veröffentlichungstag der Anmeldung: 02.12.2009
(73) Patentinhaber: Südzucker Aktiengesellschaft Mannheim/Ochsenfurt, 68165 Mannheim (DE)
(72) Erfinder: KOWALCZYK, Jörg, 67304 Eisenberg/Steinborn (DE); HAUSMANNS, Stephan, 65185 Wiesbaden (DE)
(74) Vertreter: Schrell, Andreas
(86) Internationale Anmeldenummer: PCT/EP2008/001394
(87) Internationale Veröffentlichungsnummer: WO 2008/101707

(56) Entgegenhaltungen:
- EP-A- 0 390 438
- EP-A- 1 410 721
- EP-A- 1 424 074
- EP-A- 1 548 093
- WO-A-02/096223
- WO-A-2004/047566
- WO-A-2004/057982
- WO-A-2005/013720
- WO-A-2006/007993
- US-A1- 2007 037 756
- SCHRAUWEN, P. ET AL: "Increase in Fat Oxidation on a High-Fat Diet Is Accompanied by an Increase in Triglyceride-Derived Fatty Acid Oxidation" DIABETES, Bd. 49, Nr. 4, 2000, Seiten 640-646, XP002484927

## Beschreibung

Die vorliegende Erfindung betrifft Nahrungsmittelzusammensetzungen zur Gewichts- und/oder Appetitkontrolle, umfassend mindestens zwei jeweils mindestens eine ernährungsphysiologisch positiv wirkende Funktionalität aufweisende Bestandteile, wobei der erste Bestandteil eine appetitzügelnde Funktion und der zweite Bestandteil eine niedrig-glykämische Funktionalität aufweist sowie die Verwendung niedrig-glykämischer Nahrungsmittelbestandteile in Nahrungsmittelzusammensetzungen.

Moderne Nahrungsmittel müssen heutzutage verschiedenen Anforderungen gerecht werden. Neben ihrer primären Funktion als Energieträger kommt ihnen in jüngster Zeit insbesondere auch eine gesundheitserhaltende und gesundheitsfördernde Funktionalität zu. Nahrungsmittel sollen gut schmecken, dabei die notwendige Energiezufuhr sicherstellen, gleichzeitig aber auch einer unerwünschten Gewichtszunahme mit den damit verbundenen negativen gesundheitlichen Folgen entgegenwirken.

So ist aus US 6,025,008 ein probiotisches Joghurt bekannt, das aufbereitetes Fischöl zusammen mit einem Süßungsmittel, beispielsweise Isomaltulose, Maltose, Trehalose oder Maltit enthält. Das Joghurt ist ernährungsphysiologisch deshalb vorteilhaft, weil es aufgrund seines Gehaltes an ungesättigten Fettsäuren sowohl der Prophylaxe als auch der Therapie von kardiovaskulären Erkrankungen dienen kann. Die eingesetzten Süßungsmittel maskieren dabei den vielfach unerwünschten Fischgeruch.

Aus der WO 03/022288 A1 gehen Nahrungsmittelzusammensetzungen hervor, die Lipide und Kohlenhydrate enthalten, wobei als Kohlenhydrat Palatinose oder Trehalulose eingesetzt sein kann. Die dort beschriebenen Nahrungsmittelzusammensetzungen erweisen sich ernährungsphysiologisch als sinnvoll für Diabetes-Patienten ebenso wie zur Verhinderung von Fettleibigkeit.

Aus der WO 2004/057982 geht die Verwendung von bestimmten Triacylglycerolen in Lebensmitteln zur Erzielung eines Sättigungsgefühls hervor.

Aus der EP-A-1 548 093 geht ein Sirup hervor, der Isomaltulose und eine DHA-haltige Öl-in-Wasser-Emulsion enthält. Die EP-A-0 390 438 offenbart Isomaltulose-haltige Süßstoff-Zusammensetzungen für Getränke, Konfekt und Backwaren.

Nach wie vor besteht jedoch ein weiterer Bedarf an Nahrungsmitteln, die vorteilhafte ernährungsphysiologische und technologische Eigenschaften, zum Beispiel eine erhöhte Stabilität, mit sich bringen. Insbesondere ist es wünschenswert, eine lagerstabile Nahrungsmittelzusammensetzung zur Verfügung zu stellen, die einerseits gut schmeckt und die die für eine Gesunderhaltung des Konsumenten notwendige Nahrungsmittelbestandteile enthält, andererseits aber auch den Konsumenten nicht zu einem übermäßigen Verzehr und damit letztlich zu Fettleibigkeit verleitet, wobei diese Nahrungsmittelzusammensetzung gleichzeitig vergleichsweise einfach und kostengünstig hergestellt werden kann.

Die vorliegende Erfindung löst das ihr zugrundeliegende technische Problem durch die Bereitstellung einer Nahrungsmittelzusammensetzung zur Gewichts- und/oder Appetitkontrolle gemäß Anspruch 1, umfassend mindestens zwei jeweils mindestens eine ernährungsphysiologisch positiv wirkende Funktionalität aufweisende Bestandteile, wobei der erste Bestandteil eine appetitzügelnde Funktionalität und der zweite Bestandteil eine niedrig-glykämische Funktionalität aufweist, wobei beide Bestandteile in einer ihre ernährungsphysiologisch positive Funktionalität entfaltenden Menge in der Nahrungsmittelzusammensetzung vorhanden sind und wobei der erste Bestandteil eine Öl-in-Wasser-Emulsion mit einem Gehalt an Triacylglycerolbeziehungsweise Triglyceridölen aufweist. In einer besonders bevorzugten Ausführungsform sind die in der Ölphase befindlichen Triglyceridöle so ausgestaltet, dass sie bei Raum- bis Körpertemperatur des gesunden menschlichen Körpers in fester Form vorliegen. Derartige Triglyceridöle sind Palmöl oder Kakaobutter beziehungsweise Fraktionen davon, wobei diese in bevorzugter Ausführungsform bei Raum- bis Körpertemperatur des Konsumenten in fester Form vorliegen sollten. Vorzugsweise weisen die Triglyceridöle wenigstens 90, vorzugsweise wenigstens 95 Gew.-%, besonders bevorzugt mindestens 99 Gew.-% Triglyceride (bezogen auf Gesamtgewicht des Triglyceridöls), auf. Die Triglyceride sind vorzugsweise Glycerolester von Linolsäure, Oleinsäure, Palmitin- oder Stearinsäure.

Die erfindungsgemäß eingesetzte Emulsion mit einem Gehalt an Triglyceridölen weist neben den in der Emulsion vorhandenen Triglyceridölen auch mindestens einen Emulgator auf, der vorzugsweise nahrungsmittelverträglich ist, und der die Triglyceridöle in der wässrigen Phase in Emulsion hält. Gemäß der Erfindung handelt es sich um einen Galactolipid-basierten Emulgator, zum Beispiel Mono- oder Digalactosyldiglyceride oder um Lecithin. Die Menge und Art des Emulgators ist erfindungsgemäß so einzustellen, dass die Triglyceridöle, die sich in der Emulsion befinden, in Emulsion gehalten werden können, insbesondere bei Raum- bis Körpertemperatur des Konsumenten.

In besonders bevorzugter Auführungsform der vorliegenden Erfindung ist das Galactolipid Haferöl, insbesondere fraktioniertes Haferöl.

In einer weiteren bevorzugten Ausführungsform ist vorgesehen, dass die Ölphase der Emulsion 80 bis 99 Gew.-% Triglyceridöle und 1 bis 20 Gew.-% lebensmittelverträglichen Emulgator (jeweils bezogen auf Gewicht der Ölphase) enthält.

In einer weiteren bevorzugten Ausführungsform kann vorgesehen sein, dass die Triglyceridöle zusammen mit Kokosnuss- oder Palmkernöl vorliegen.

In einer besonders bevorzugten Ausführungsform kann vorgesehen sein, dass die wässrige Komponente der Emulsion Wasser ist, wobei dieses zusätzlich mit Geschmacksstoffen, Farbstoffen, Aromastoffen, Süßungsmitteln, Verdickungsmitteln, Konservierungsmitteln, Antioxidanzien, Mineralien, Spurenelementen etc. versehen sein kann.

In einer besonders bevorzugten Ausführungsform der vorliegenden Erfindung kann die wässrige Phase der Emulsion selbst den zweiten Bestandteil der erfindungsgemäßen Nahrungsmittelzusammensetzung, das heißt das niedrig-glykämische Saccharoseisomer, also Isomaltulose und/oder Leukrose, enthalten.

In besonders bevorzugter Ausführungsform ist vorgesehen, dass der Gesamtfettgehalt der Emulsion 40 bis 50 Gew.-% (bezogen auf Gesamtgewicht der Emulsion) beträgt.

Im Zusammenhang mit der vorliegenden Erfindung wird unter einem niedrig-glykämischen Saccharoseisomer ein Isomer, das heißt ein Strukturisomer von Saccharose verstanden, welches als niedrig-glykämisches Kohlenhydrat im menschlichen Körper wirkt, das heißt nach Konsum in einem vergleichsweise niedrigen respiratorischen Quotienten resultiert. Der respiratorische Quotient spiegelt das Verhältnis von CO₂/O₂ in der Atemluft wieder und ist ein Maß dafür, welche Nährstoffe verbrannt werden. Reine Kohlenhydratverbrennung führt zu einem respiratorischen Quotienten von 1, während reine Fettverbrennung zu einem respriratorischen Quotienten von 0,7 führt. Niedrig-glykämische Kohlenhydrate unterstützen somit passiv die Nutzung von Fett zur Energiegewinnung im Körper, da sie den Insulinspiegel nicht sehr stark ansteigen lassen und damit die Fettoxidation begünstigen.

Im Zusammenhang mit der vorliegenden Erfindung werden unter niedrig-glykämischen Saccharoseisomeren insbesondere Leukrose und Isomaltulose verstanden. Der Begriff "niedrig-glykämisches Saccharoseisomer" im Rahmen der vorliegenden Erfindung steht daher insbesondere dafür, dass in der erfindungsgemäßen Nahrungsmittelzusammensetzung als niedrig-glykämisches Kohlenhydrat a) Isomaltulose oder b) Leukrose oder c) Leukrose und Isomaltulose, vorzugsweise in einem Verhältnis von 1:99 Gew.-% bis 99:1 Gew.-%, vorzugsweise 30:70 Gew.-% zu 70:30 Gew.-% eingesetzt werden (jeweils bezogen auf Gesamt-Trockensubstanzgehalt der beiden Isomere).

Der erste Bestandteil der erfindungsgemäßen Nahrungsmittelzusammensetzung bewirkt ein verstärktes, schnell einsetzendes und verlängertes Sättigungsgefühl im Konsumenten und weist daher appetitzügelnde und damit gewichtskontrollierende Funktionalität auf.

Die Kombination des appetizügelnden ersten Bestandteils mit dem zweiten Bestandteil, also dem niedrig-glykämischen Saccharoseisomer, also Isomaltulose und/oder Leukrose, führt überraschenderweise zu einer besonders lagerstabilen Zusammensetzung, insbesondere mit besonders positiven ernährungsphysiologischen Funktionalitäten. Zu diesen ernährungsphysiologisch positiven Funktionalitäten des ersten Bestandteils zählt die durch die efindungsgemäße Nahrungsmittelzusammensetzung bewirkte schnell einsetzende Sättigung des Konsumenten, die frühzeitig zu einem nachhaltigen, lange währenden Abklingen von Hungergefühl führt und damit der Prophylaxe und Behandlung von Fettleibigkeit, der Kontrolle von Kalorien- und Fettaufnahme dient und so letztendlich auch kardiovaskulären Krankheiten sowie Diabetes vorbeugt. Zu den ernährungsphysiologisch positiven Funktionalitäten des zweiten Bestandteils zählt dessen Eigenschaft, im Verdauungstrakt des Konsumenten verlangsamt Glucose freizusetzen, dessen Eigenschaft, nicht zu einem raschen Anstieg des Blutglucosespiegels zu führen und dessen Eigenschaft niedrig-glykämisch zu sein, und damit zu einem geringeren Anstieg des Insulinspiegels zu führen mit der Folge, die Fettoxidation zu begünstigen. Andererseits konnte überraschenderweise beobachtet werden, dass die Stabilität des fetthaltigen ersten Bestandteils in einer Nahrungsmittelzusammensetzung in Anwesenheit von mindestens einem niedrig-glykämischen Saccharoseisomer, also von Isomaltulose, Leukrose oder einer Kombination von Isomaltulose und Leukrose, erheblich erhöht wurde, insbesondere die Stabilität einer oberflächenvergrößerten Flocken- oder Aggregatform, in der sich die Bestandteile der erfindungsgemäßen Zusammensetzung in bevorzugter Ausführung befinden. Schließlich zeigt sich aufgrund der Anwesenheit des zweiten Bestandteils eine verbesserte Verarbeitbarkeit des ersten Bestandteils im Verlauf der Herstellung von verschiedenen Nahrungsmitteln.

In einer besonders bevorzugten Ausführungsform betrifft die vorliegende Erfindung eine vorgenannte Nahrungsmittelzusammensetzung, wobei diese Nahrungsmittelzusammensetzung 1 bis 50 Gew.-% des ersten Bestandteils, vorzugsweise 2 bis 50 Gew.-%, vorzugsweise 2 bis 15 Gew.-%, insbesondere 3 bis 10 Gew.-%, vorzugsweise 3 bis 50 Gew.-%, insbesondere 4 bis 40 Gew.-% und insbesondere 5 bis 30 Gew.-% des ersten Bestandteils enthält (jeweils bezogen auf Gesamttrockensubstanz der Nahrungsmittelzusammensetzung).

In einer weiteren bevorzugten Ausführungsform betrifft die Erfindung eine vorgenannte Nahrungsmittelzusammensetzung, wobei diese Nahrungsmittelzusammensetzung vorzugsweise 1 bis 50 Gew.-% niedrig-glykämisches Saccharoseisomer, insbesondere 2 bis 50 Gew.-%, vorzugsweise 2 bis 15 Gew.-%, insbesondere 3 bis 10 Gew.-%, insbesondere 3 bis 50 Gew.%, insbesondere 4 bis 40 Gew.-% oder 5 bis 30 Gew.-% niedrig-glykämisches Saccharoseisomer enthält (jeweils bezogen auf Gesamttrockensubstanz der Nahrungsmittelzusammensetzung).

Die vorliegende Erfindung betrifft insbesondere auch eine der vorgenannten Nahrungsmittelzusammensetzungen, enthaltend 1, 2, 3, 4, 5, 6, 7, 8, 9 oder 10 Gew.-% bis 10, 15, 20, 30, 40 oder 50 Gew.-% ersten Bestandteil und 1, 2, 3, 4, 5, 6, 7, 8, 9 oder 10 Gew.-% bis 10, 15, 20, 30, 40 oder 50 Gew.-% niedrig-glykämisches Saccharoseisomer (jeweils bezogen auf Gesamttrockensubstanz der Nahrungsmittelzusammensetzung).

In einer weiteren bevorzugten Ausführungsform weist die Nahrungsmittelzusammensetzung der vorliegenden Erfindung neben dem ersten Bestandteil und dem mindestens einen niedrig-glykämischen Saccharoseisomer mindestens einen, vorzugsweise mehrere Zusatzstoff(e) auf, insbesondere in einer auf 100 Gew.-% Gesamttrockensubstanz aufaddierenden Menge, zum Beispiel in einer Menge von 1 bis 99 Gew.-%, vorzugsweise 40 bis 90 Gew.-%, vorzugsweise 2 bis 85 Gew.-%, 3 bis 70 Gew.-%, 5 bis 60 Gew.-%, 10 bis 50 Gew.-%, 20 bis 40 Gew.-%, 30 bis 80 Gew.-%, 40 bis 70 Gew.-%, 45 bis 60 Gew.-% oder 25 bis 38 Gew.%, bezogen auf das Gesamttrockengewicht der Nahrungsmittelzusammensetzung.

Unter einem Zusatzstoff werden solche Stoffe verstanden, die insbesondere das Aussehen, den Geschmack, die Organoleptik, den Nährwert, ernährungsphysiologische Eigenschaften, die Verarbeitbarkeit, die Lagerfähigkeit oder die Gebrauchsfertigkeit der Nahrungsmittelzusammensetzung beeinflussen.

In einer weiteren bevorzugten Ausführungsform ist vorgesehen, dass der mindestens eine Zusatzstoff ein Präbiotikum, ein Probiotikum, ein Ergänzungsstoff, eine fetthaltige Komponente, ein Milcherzeugnis oder ein süßendes Agens ist.

Im Zusammenhang mit der vorliegenden Erfindung werden unter dem Begriff "süßendes Agens" Substanzen verstanden, die Süßkraft aufweisen und zum Beispiel Lebensmitteln oder Getränken zugesetzt werden, um einen Süßegeschmack hervorzurufen. Im Zusammenhang mit der vorliegenden Erfindung werden die "süßenden Agenzien" unterteilt in "Zucker" wie Isomaltulose, Saccharose, Glucose oder Fructose, die Körper und Süßkraft geben sowie "Süßungsmittel", also Stoffe, die keine Zucker sind, aber trotzdem Süßkraft aufweisen, welche wiederum untergliedert werden in "Zuckeraustauschstoffe", also süßende Agenzien, die einen Körper und einen physiologischen Brennwert zusätzlich zu einer Süßkraft aufweisen (körpergebende Süßungsmittel), und "Intensivsüßstoffe", also Stoffe, die in der Regel eine sehr hohe Süßkraft, aber keinen Körper und in der Regel keinen oder nur einen geringfügigen physiologischen Brennwert aufweisen. Ein Intensivsüßstoff ist beispielsweise Cyclamat, Acesulfam H, Aspartam oder Sucralose.

In einer besonders bevorzugten Ausführungsform ist daher das sü-βende Agens ein Zucker, ein Intensivsüßstoff oder ein Zuckeraustauschstoff.

In einer weiteren bevorzugten Ausführungsform ist der Intensivsüßstoff ausgewählt aus der Gruppe bestehend aus Sucralose, Natriumcyclamat, Acesulfam K, Neohesperidin-Dihydrochalkon, Glycyrrhizin, Steveosid, Monellin, Thaumatin, Aspartam, Dulcin, Saccharin, Naringin-Dihydrochalkon, Neotame und einer Mischung zweier oder mehrerer davon. In einer weiteren bevorzugten Ausführungsform ist der Zuckeraustauschstoff ausgewählt aus der Gruppe bestehend aus Isomalt, 1,1-GPM (1-O-α-D-Glucopyranosyl-D-mannit), 1,6-GPS (6-O-α-D-Glucopyranosyl-D-sorbit), 1,1-GPS (1-O-α-D-Glucopyranosyl-D-sorbit), Maltodextrine, Lactit, Maltit, Erythrit, Xylit, Mannit, Sorbit, Maltitsirup, hydrierte und nichthydrierte Stärkehydrolysate und einer Mischung zweier oder mehrerer davon.

In einer weiteren bevorzugten Ausführungsform ist der mindestens eine Zusatzstoff ein Zucker, insbesondere Saccharose, Glukose, Fructose, Lactose, Maltose oder ein Gemisch zweier oder mehrerer davon.

Selbstverständlich betrifft die Efindung auch Nahrungsmittelzusammensetzungen enthaltend den ersten Bestandteil und das mindestens eine niedrig-glykämische Saccharoseisomer, wobei das mindestens eine niedrig-glykämische Saccharoseisomer das einzig und alleinig in der Nahrungsmittelzusammensetzung vorkommende körpergebende süßende Agens ist. In einer weiteren bevorzugten Ausführungsform betrifft die Erfindung eine vorgenannte Nahrungsmittelzusammensetzung, enthaltend den ersten Bestandteil und das mindestens eine niedrig-glykämische Saccharoseisomer, wobei das mindestens eine niedrig-glykämische Saccharoseisomer der einzig und alleinige in der Nahrungsmittelzusammensetzung vorkommende Zucker ist. In einer weiteren bevorzugten Ausführungsform betrifft die vorliegende Erfindung eine Nahrungsmittelzusammensetzung enthaltend den ersten Bestandteil und das mindestens eine niedrig-glykämische Saccharoseisomer, wobei das mindestens eine niedrig-glykämische Saccharoseisomer das einzig und alleinig in der Nahrungsmittelzusammensetzung vorkommende süßende Agens ist.

Insbesondere betrifft die Erfindung eine Nahrungsmittelzusammensetzung enthaltend oder bestehend aus dem ersten Bestandteil und das beziehungsweise dem mindestens eine beziehungsweise einem niedrig-glykämische/en Saccharoseisomer, wobei, optional, die Nahrungsmittelzusammensetzung diabetikergeeignet oder diätetisch ist.

In einer weiteren bevorzugten Ausführungsform betrifft die vorliegende Erfindung eine vorliegende Nahrungsmittelzusammensetzung, enthaltend den ersten Bestandteil und das mindestens eine niedrig-glykämische Saccharoseisomer, wobei diese Nahrungsmittelzusammensetzung frei ist von Saccharose, frei ist von Glucose, frei ist von Lactose, frei ist von Fructose, frei ist von Sorbit, frei ist von Xylit, frei ist von Mannit oder frei ist von einem oder mehreren oder allen der genannten Zucker oder Zuckeralkohole.

In einer weiteren bevorzugten Ausführungsform betrifft die vorliegende Erfindung eine Nahrungsmittelzusammensetzung, enthaltend den ersten Bestandteil und das mindestens eine niedrig-glykämische Saccharoseisomer, wobei diese als Zusatzstoff ein Präbiotikum, insbesondere Inulin, Oligofructose, Galactooligosaccharide enthält.

In einer weiteren bevorzugten Ausführungsform betrifft die vorliegende Erfindung eine Nahrungsmittelzusammensetzung, enthaltend den ersten Bestandteil, einen Zusatzstoff und das mindestens eine niedrig-glykämische Saccharoseisomer, wobei der mindestens eine Zusatzstoff ein Milcherzeugnis, insbesondere ein lactosefreies Milcherzeugnis ist, zum Beispiel Magermilchpulver, Vollmilchpulver, lactosefreies Magermilchpulver, lactosefreies Vollmilchpulver, ein Molkeerzeugnis oder eine Mischung zweier oder mehrerer davon.

In einer weiteren bevorzugten Ausführungsform betrifft die vorliegende Erfindung eine Nahrungsmittelzusammensetzung, enthaltend den ersten Bestandteil, einen Zusatzstoff und das mindestens eine niedrig-glykämische Saccharoseisomer, wobei der mindestens eine Zusatzstoff ein Ergänzungsstoff ist ausgewählt aus der Gruppe bestehend aus Malzextrakt, Aromastoffen, Farbstoffen, Konservierungsstoffen, Trennmitteln, Aromen, Geschmacksstoffen, Fließmittel, Mineralstoffen wie Natrium und Kalzium, insbesondere Salzen wie Natriumchlorid, Vitaminen, Folsäure, Emulatoren, Ballaststoffen, Lecithin, Omega-3-Fettsäuren, Triglyzeride mittlerer Kettenlänge, Phythoöstrogene und Ascorbinsäuresalze.

In einer weiteren bevorzugten Ausführungsform betrifft die Erfindung eine Nahrungsmittelzusammensetzung, enthaltend den ersten Bestandteil, einen Zusatzstoff und das mindestens eine niedrig-glykämische Saccharoseisomer, wobei als Zusatzstoff ein Probiotikum, zum Beispiel Lactobakterien oder Bifidobakterien vorhanden sind.

In einer weiteren bevorzugten Ausführungsform ist die Nahrungsmittelzusammensetzung der vorliegenden Erfindung ein Produkt, welches als Nahrungs-, Genuss- oder Arzneimittel ausgeführt ist, vorzugsweise als diätetisches Nahrungs- oder Genussmittel.

Erfindungsgemäß bevorzugt weist das Nahrungs-, Genuss- oder Arzneimittel 1 bis 99 Gew.-%, 2 bis 99 Gew.-%, insbesondere 20 bis 70 Gew.-%, bevorzugt 30 bis 60 Gew.-%, mehr bevorzugt 40 bis 55 Gew.-%, (bezogen auf die Gesamt-Trockensubstanz des Nahrungs-, Genuss- oder Arzneimittels) des ersten und zweiten Bestandteils auf. In besonderer Ausführungsform ist das Nahrungs-, Genuss- oder Arzneimittel frei von Glucose, Fructose, Lactose, Saccharose, Sorbit, Xylit und/oder Mannit. Erfindungsgemäß kann es jedoch auch Glucose, Fructose, Saccharose und/oder andere süßende Agenzien enthalten.

Im Zusammenhang mit der vorliegenden Erfindung werden unter dem Begriff "Nahrungsmittel" vorwiegend der menschlichen Ernährung dienende Erzeugnisse oder Stoffgemische in fester, halbfester, flüssiger, gelöster oder suspendierter Form verstanden, die dazu vorwiegend bestimmt sind, in unverändertem, zubereitetem oder verarbeitetem Zustand vom Menschen verzehrt zu werden. Nahrungsmittel können neben ihren natürlichen Bestandteilen weitere Komponenten enthalten, die natürlicher oder synthetischer Herkunft sein können. Nahrungsmittel können sowohl in fester Form als auch in flüssiger Form vorliegen. Unter einem Genussmittel" werden vorwiegend dem beim Verzehr auftretenden Genuss des menschlichen oder tierischen Körpers dienende Stoffe oder Stoffgemische in fester, halbfester, flüssiger, gelöster oder suspendierter Form verstanden.

In bevorzugter Ausführungsform der Erfindung handelt es sich bei den erfindungsgemäßen Nahrungsmitteln um Milcherzeugnisse oder Milchprodukte, beispielsweise Käse-, Butter-, Joghurt-, Eiscreme-, Trinkjoghurt-, Kefir-, Quark, Sauermilch-, Buttermilch-, Sahne-, Kondensmilch-, Trockenmilch-, Molken-, Milchmisch-, Milchhalbfett-, Molkenmisch- Milchzucker-, Milcheiweiß- und Milchfett-Produkte. In einer weiteren bevorzugten Ausführungsform der Erfindung handelt es sich bei den erfindungsgemäßen Nahrungsmitteln um Backwaren, insbesondere Brot einschließlich Kleingebäck und feine Backwaren einschließlich Dauer-Backwaren. In weiteren Ausführungsformen der Erfindung handelt es sich bei den erfindungsgemäßen Nahrungsmitteln um Brotaufstriche, Margarine-Erzeugnisse und Backfette sowie Instantprodukte und Brüherzeugnisse. Ein Nahrungsmittel im Sinne der vorliegenden Erfindung kann auch ein Getränkepulver, zum Beispiel ein Instantgetränkepulver sein, zum Beispiel eine Kakao-, Tee- oder Kaffeprodukt-Getränkepulver.

In einer besonders bevorzugten Ausführungsform der vorliegenden Erfindung ist das Nahrungsmittel ein flüssiges Nahrungsmittel, also ein Getränk, insbesondere ein Getränk auf Basis von Wasser oder Milch. Die Getränke können alkoholische oder nicht-alkoholische Getränke sein. Die Getränke können in einer bevorzugten Ausführungsform diabetiker-geeignet sein. In besonders bevorzugter Ausführungsform ist die Nahrungsmittelzusammensetzung ein Getränk, zum Beispiel ein Milchgetränk, ein Milchmischgetränk, Sportlergetränk, ein Energiegetränk, eine enterale Formulierung, ein Erfrischungsgetränk, ein Cola-haltiges Getränk oder ähnliches.

Unter dem Begriff Genussmittel werden beispielsweise feste oder halbfeste Süßwaren, insbesondere Schokoladen-Erzeugnisse, Hartkaramellen, Weichkaramellen, Fondant-Erzeugnisse, Gelee-Erzeugnisse, Lakritzen, Schaumzuckerwaren, Kokosflocken, Dragees, Komprimate, kandierte Früchte, Krokant, Nougaterzeugnisse, Eiskonfekt, Marzipan, Kaugummi, Müsliriegel, sowie Speiseeis verstanden.

Im Zusammenhang mit der vorliegenden Erfindung werden unter "diätetischen Nahrungsmitteln beziehungsweise Getränken" Nahrungsmittel beziehungsweise Getränke verstanden, die bestimmt sind, einem bestimmten Ernährungszweck dazu zu dienen, dass sie die Zufuhr bestimmter Nährstoffe oder anderer ernährungsphysiologisch wirkender Stoffe in einem bestimmten Mengenverhältnis oder in bestimmter Beschaffenheit bewirken. Diätetische Nahrungsmittel oder Getränke unterscheiden sich maßgeblich von Nahrungsmitteln oder Getränken vergleichbarer Art durch ihre Zusammensetzung oder durch ihre Eigenschaften. Diätetische Nahrungsmittel können in Fällen eingesetzt werden, wo bestimmte Ernährungsanforderungen aufgrund von Krankheiten, Funktionsstörungen oder allergischen Reaktionen gegen einzelne Nahrungsmittel beziehungsweise deren Inhaltsstoffe erfüllt werden müssen. Diätetische Nahrungsmittel können in fester, halbfester und in flüssiger Form (Getränk) vorliegen.

Die Erfindung betrifft auch Verfahren zur Herstellung der vorgenannten Nahrungsmittelzusammensetzungen. Erfindungsgemäß ist daher vorgesehen, ein Verfahren zur Herstellung der vorgenannten Nahrungsmittelzusammensetzungen bereitzustellen, in dem der erste Bestandteil und der zweite Bestandteil, insbesondere das mindestens eine niedrig-glykämische Saccharoseisomer bereitgestellt und miteinander im gewünschten Verhältnis, gegebenenfalls zusammen mit dem mindestens einen Zusatzstoff, vermischt werden.

Offenbart wird auch die Verwendung von dem mindestens einem niedrig-glykämischen Saccharoseisomer, insbesondere Isomaltulose und/oder Leukrose, als niedrig-glykämischer Bestandteil in einem eine Triglyceridöl-haltige Emulsion enthaltenden Nahrungs-, Genuss- oder Arzneimittel zur Gewichtskontrolle, zur Appetitkontrolle, zur Kontrolle der Fettaufnahme, zur Kontrolle der Kalorienaufnahme, zur Verbesserung der Fett- und/oder Kalorienverwertung, zur Prophylaxe oder Therapie von Fettleibigkeit, Fettsucht, Diabetes, cardiovaskulären Erkrankungen oder dergleichen.

Die Erfindung betrifft auch die Verwendung von mindestens einem niedrig-glykämischen Saccharoseisomer, insbesondere Isomaltulose und/oder Leukrose, in einem eine Triglyceridöl-haltige Emulsion enthaltenden Nahrungs-, Genuss- oder Arzneimittel zur Erhöhung der Stabilität der Emulsion in dem Nahrungs-, Genuss- oder Arnzeimittel.

Weitere vorteilhafte Ausgestaltungen der Erfindung ergeben sich aus den Unteransprüchen.

Die Erfindung wird anhand des beigefügten Beispiels näher erläutert.

### Beispiel

Als Nachweis zur sättigenden Wirkung einer Isomaltulose (und Leucrose)-Palmkernöl-Mischung wird anhand von in vitro-Versuchen die verbesserte Stabilität und somit ein verbessertes Eigenschaftsprofil bei Verwendung von Isomaltulose bzw. Leucrose als Stabilitätsverbesserer gezeigt.

Als Rohstoff wurde als Beispiel für das Triglycerid Palmkernöl und als Emulgator Lecithin eingesetzt. Es wurden Versuche durchgeführt, die nur diese beiden Komponenten verwendeten sowie Versuche mit unterschiedlichen Konzentrationen von Kohlenhydratzusätzen. Neben Isomaltulose und Leucrose wurde zur Kontrolle auch Saccharose getestet.

Die Herstellung der Emulsionen erfolgte durch Hochdruckemulgierung, wobei nach einem ersten Screening gezeigt werden konnte, dass bereits bei einem Druck von 700 bar visuell stabile Emulsionen erhalten werden. Die Durchführung der Hochdruckemulgierung erfolgte letztendlich bei Drücken von 790 - 810 bar.

Anschließend wurden in 200 ml eines synthetischen Magensaftes (für 1000 ml werden 3,25 g Pepsin, 2,02 g NaCl, in 500 ml Wasser gelöst, mit 1 M HCl wird auf pH 1 eingestellt und anschließend mit destilliertem Wasser aufgefüllt) 10 g der durch Hochdruckemulgierung gewonnenen Produkte bei 37°C unter Rühren eingebracht und in Abhängigkeit von der Zeit die Stabilität bzw. das Demulgierverhalten (freiwerden von Öl) beobachtet. Um einen Effekt bezüglich der sättigenden Wirkung auch aus physikalisch chemischen Gesichtpunkten ableiten zu können, sollten die entstehenden Dispersionen möglichst voluminös sein, also eine große Oberfläche aufweisen und möglichst bis zu einer Stunde stabil sein. Dieses konnte eindeutig für die beiden reduzierenden Disaccharide Isomaltulose und Leucrose belegt werden.

Rezepturen der Versuchsemulsionen A-F(Angaben in Gew.%):

| **Versuch** | **A** | **B** | **C** | **D** | **E** |
|---|---|---|---|---|---|
| Palmkernöl | 40 | 40 | 40 | 40 | 40 |
| Lecithin | 2 | 2 | 2 | 2 | 2 |
| Saccharose | - | 3 | - | - | - |
| Isomaltulose | - | - | 2 | 6 | - |
| Leucrose | - | - | - | - | 2 |
| Dest. Wasser | 58 | 55 | 56 | 55 | 56 |

Ergebnisse nach Dispergierung im synthetischen Magensaft:
Folgende Abstufung der Beurteilung bezüglich der Stabilität wurde herangezogen:
++ voluminöse stabile Flocken an der Oberfläche
+ leicht aggregierte Flocken an der Oberfläche, keine Demulgierung
- leicht aggregierte Flocken an der Oberfläche, einsetzende Demulgierung (Öltröpfchen)
-- aggregierte Flocken, deutliche Demulgierung (Ölseparation)

| **Zeit in Minuten** | **A** | **B** | **C** | **D** | **E** |
|---|---|---|---|---|---|
| 1-2 | ++ | ++ | ++ | ++ | ++ |
| 15 | ++ | ++ | ++ | ++ | ++ |
| 30 | - | + | ++ | ++ | ++ |
| 45 | -- | - | ++ | ++ | ++ |
| 60 | | -- | + | + | + |

Die Ergebnisse belegen, dass die Verwendung von Isomaltulose und Leucrose in den Palmkernöl/Lecithin-Gemischen einen deutlich positiven Einfluss auf die Stabilität hat, insbesondere eine verbesserte, das heisst erhöhte Stabilität der sich bildenden oberflächenvergrö-βerten Teilchen in Flockenform bewirkt.

## Patentansprüche

1. Nahrungsmittelzusammensetzung zur Gewichts- und/oder Appetitkontrolle, umfassend mindestens zwei jeweils mindestens eine ernährungsphysiologisch positiv wirkende Funktionalität aufweisende Bestandteile, wobei der erste Bestandteil eine appetitzügelnde und der zweite Bestandteil eine niedrig-glykämische Funktionalität aufweist, wobei beide Bestandteile in einer ihre ernährungsphysiologisch positive Wirkung entfaltenden Menge in der Nahrungsmittelzusammensetzung vorhanden sind, wobei der erste Bestandteil eine Emulsion mit einem Gehalt an Triglyceridölen, wobei die Triglyceridöle Palmöl oder Kakaobutter sind, und der zweite Bestandteil Isomaltulose oder Leukrose ist, und wobei die Emulsion eine Öl-in-Wasser-Emulsion aus Triglyceridölen mit einem nahrungsmittelverträglichen Emulgator in wässriger Lösung ist, wobei der nahrungsmittelverträgliche Emulgator ein Galactolipid oder Lecithin ist.

2. Nahrungsmittelzusammensetzung nach Anspruch 1, wobei die Triglyceridöle fraktioniertes Palmöl ist.

3. Nahrungsmittelzusammensetzung nach Anspruch 1 oder 2, wobei das Galactolipid Haferöl, insbesondere fraktioniertes Haferöl ist.

4. Nahrungsmittelzusammensetzung nach einem der vorhergehenden Ansprüche, wobei die Ölphase der Emulsion 80 bis 99 Gew.-% Triglyceridöle und 1 bis 20 Gew.-% nahrungsmittelverträglichen Emulgator (jeweils bezogen auf die Ölphase) enthält.

5. Nahrungsmittelzusammensetzung nach einem der vorhergehenden Ansprüche, wobei die Triglyceridöle mindestens 90 Gew.-% Triglyceride enthalten.

6. Nahrungsmittelzusammensetzung nach einem der vorhergehenden Ansprüche, wobei die Triglyceridöle zusammen mit Kokosnuss- oder Palmkernöl vorliegen.

7. Nahrungsmittelzusammensetzung nach einem der vorhergehenden Ansprüche, wobei der Gesamtfettgehalt der Emulsion 40 bis 50 Gew.-% (bezogen auf Gesamtgewicht der Emulsion) beträgt.

8. Nahrungsmittelzusammensetzung nach einem der vorhergehenden Ansprüche, wobei die Nahrungsmittelzusammensetzung 1 bis 50 Gew.-% (bezogen auf Gesamttrockensubstanz der Nahrungsmittelzusammensetzung) des ersten Bestandteils enthält.

9. Nahrungsmittelzusammensetzung nach einem der vorhergehenden Ansprüche, wobei die Nahrungsmittelzusammensetzung 1 bis 50 Gew.-% des zweiten Bestandteils (bezogen auf Gesamttrockensubstanz der Nahrungsmittelzusammensetzung) enthält.

10. Nahrungsmittelzusammensetzung nach Anspruch 8, wobei die Nahrungsmittelzusammensetzung 5 bis 30 Gew.-% des ersten Bestandteils (bezogen auf Gesamttrockensubstanz der Nahrungsmittelzusammensetzung) enthält.

11. Nahrungsmittelzusammensetzung nach Anspruch 9, wobei die Nahrungsmittelzusammensetzung 5 bis 30 Gew.-% des zweiten Bestandteils (bezogen auf Gesamttrockensubstanz der Nahrungsmittelzusammensetzung) enthält.

12. Nahrungsmittelzusammensetzung nach einem der vorhergehenden Ansprüche, wobei die Nahrungsmittelzusammensetzung ein festes, halbfestes oder flüssiges Nahrungsmittel, Lebensmittel oder Arzneimittel ist.

13. Nahrungsmittelzusammensetzung nach einem der vorhergehenden Ansprüche, wobei die Nahrungsmittelzusammensetzung 3 bis 50 Gew.-% des ersten Bestandteils und 3 bis 50 Gew.-% des zweiten Bestandteils (jeweils bezogen auf Gesamttrockensubstanz der Nahrungsmittelzusammensetzung) enthält.

14. Nahrungsmittelzusammensetzung nach einem der vorhergehenden Ansprüche, wobei die Nahrungsmittelzusammensetzung mindestens einen Zusatzstoff enthält.

15. Nahrungsmittelzusammensetzung nach einem der vorhergehenden Ansprüche, wobei der mindestens eine Zusatzstoff ein Präbiotikum, ein Probiotikum, ein Ergänzungsstoff, eine fetthaltige Komponente, ein Milcherzeugnis oder ein süßendes Agens ist.

16. Nahrungsmittelzusammensetzung nach einem der vorhergehenden Ansprüche, wobei das süßende Agens ein Zucker, ein Intensivsüßstoff oder ein Zuckeraustauschstoff ist.

17. Nahrungsmittelzusammensetzung nach einem der vorhergehenden Ansprüche, wobei das Präbiotikum Inulin, Oligofructose und/oder Galactooligosaccharide ist (sind).

18. Nahrungsmittelzusammensetzung nach einem der vorhergehenden Ansprüche, wobei der Intensivsüßstoff ausgewählt ist aus der Gruppe bestehend aus Sucralose, Natriumcyclamat, Acesulfam K, Neohesperidin-Dihydrochalkon, Glycyrrhizin, Steveosid, Monellin, Thaumatin, Aspartam, Dulcin, Saccharin, Naringin-Dihydrochalkon, Neotame und einer Mischung zweier oder mehrerer davon.

19. Nahrungsmittelzusammensetzung nach einem der vorhergehenden Ansprüche, wobei der Zuckeraustauschstoff ausgewählt ist aus der Gruppe bestehend aus Isomalt, 1,1-GPM (1-O-α-D-Glucopyranosyl-D-mannit), 1,6-GPS (6-O-α-D-Glucopyranosyl-D-sorbit), 1,1-GPS (1-O-α-D-Glucopyranosyl-D-sorbit), Maltodextrine, Lactit, Maltit, Erythrit, Xylit, Mannit, Sorbit, Maltitsirup, hydrierte und nichthydrierte Stärkehydrolysate und einer Mischung zweier oder mehrerer davon.

20. Nahrungsmittelzusammensetzung nach einem der vorhergehenden Ansprüche, wobei der mindestens eine Zusatzstoff ein Zucker, insbesondere Saccharose, Glucose, Fructose, Lactose, Maltose oder eine Mischung zweier oder mehrerer davon ist.

21. Nahrungsmittelzusammensetzung nach einem der vorhergehenden Ansprüche, wobei der mindestens eine Zusatzstoff ein Milcherzeugnis, insbesondere ein Lactose-freies Milcherzeugnis ist.

22. Nahrungsmittelzusammensetzung nach einem der vorhergehenden Ansprüche, wobei das Milcherzeugnis Magermilchpulver, Vollmilchpulver, Lactose-freies Magermilchpulver, Lactose-freies Vollmilchpulver, ein Molkenerzeugnis oder eine Mischung zweier oder mehrerer davon ist.

23. Nahrungsmittelzusammensetzung nach einem der vorhergehenden Ansprüche, wobei der mindestens eine Zusatzstoff ein Ergänzungsstoff ist, ausgewählt ist aus der Gruppe bestehend aus Malzextrakt, Aromastoffen, Farbstoffen, Konservierungsstoffen, Aromen, Trennmitteln, Geschmacksstoffen, Fließmittel, Mineralstoffen wie Natrium und Calcium, insbesondere Salzen wie Natriumchlorid, Vitaminen, Folsäure, Emulgatoren, Ballaststoffen, Lecithin, Omega-3-Fettsäuren, Triglyzeride mittlerer Kettenlänge, Phythoöstrogene und Ascorbinsäuresalze.

24. Nahrungsmittelzusammensetzung nach einem der vorhergehenden Ansprüche, wobei das Probiotikum Lactobacillen oder Bifidobakterien sind.

25. Nahrungsmittelzusammensetzung nach einem der vorhergehenden Ansprüche, wobei die Isomaltulose oder Leukrose das einzige und alleinig in der Nahrungsmittelzusammensetzung vorkommende körpergebende süßende Agens ist.

26. Nahrungsmittelzusammensetzung nach einem der vorhergehenden Ansprüche, wobei die Isomaltulose oder Leukrose der einzige und alleinig in der Nahrungsmittelzusammensetzung vorkommende Zucker ist.

27. Nahrungsmittelzusammensetzung nach einem der vorhergehenden Ansprüche, wobei die Isomaltulose oder Leukrose das einzige und alleinig in der Nahrungsmittelzusammensetzung vorkommende süßende Agens ist.

28. Verwendung von Isomaltulose und/oder Leukrose in einem eine Triglyceridöl-haltige Emulsion enthaltenden Nahrungs-, Genuss- oder Arzneimittel zur Erhöhung der Stabilität der Emulsion in dem Nahrungs-, Genuss- oder Arzneimittel.

## Claims

1. Food composition to control weight and/or appetite, comprising at least two components, each of which showing at least one nutritionally positive-acting functionality, wherein the first component shows an appetite-depressant function and the second component a low-glycemic function, wherein both components are present in the food composition in a quantity that makes it possible to develop their nutritionally positive effect, the first component being an emulsion containing triglyceride oils, wherein said triglyceride oils are palm oil or cocoa butter, and the second component being isomaltulose or leucrose, wherein the emulsion is an oil-in-water emulsion comprised of triglyceride oils with a food-compatible emulsifying agent in an aqueous solution, wherein said food-compatible emulsifying agent is a galactolipid or lecithin.

2. Food composition according to claim 1, wherein the triglyceride oils are fractionated palm oil.

3. Food composition according to claim 1 or 2, wherein the galactolipid is oat oil, particularly fractionated oat oil.

4. Food composition according to any one of the preceding claims, wherein the oil phase of the emulsion contains 80 to 99 percent by weight of triglyceride oils and 1 to 20 percent by weight of a food-compatible emulsifying agent (in each case relative to the oil phase).

5. Food composition according to any one of the preceding claims, wherein the triglyceride oils comprise at least 90 percent by weight of triglycerides.

6. Food composition according to any one of the preceding claims, wherein the triglyceride oils are present in conjunction with cocoa nut or palm kernel oil.

7. Food composition according to any one of the preceding claims, wherein the total fat content of the emulsion amounts to 40 to 50 percent by weight (relative to the total weight of the emulsion).

8. Food composition according to any one of the preceding claims, wherein the food composition contains 1 to 50 percent by weight (relative to the total dry substance of the food composition) of the first component.

9. Food composition according to any one of the preceding claims, wherein the food composition contains 1 to 50 percent by weight of the second component (relative to the total dry substance of the food composition).

10. Food composition according to claim 8, wherein the food composition contains 5 to 30 percent by weight of the first component (relative to the total dry substance of the food composition).

11. Food composition according to claim 9, wherein the food composition contains 5 to 30 percent by weight of the second component (relative to the total dry substance of the food composition).

12. Food composition according to any one of the preceding claims, wherein the food composition is a solid, semi-solid or liquid food, food product or pharmaceutical.

13. Food composition according to any one of the preceding claims, wherein the food composition contains 3 to 50 percent by weight of the first component and 3 to 50 percent by weight of the second component (in each case relative to the total dry substance of the food composition).

14. Food composition according to any one of the preceding claims, wherein the food composition contains at least one additive.

15. Food composition according to any one of the preceding claims, wherein at least the one additive is a prebiotic, a probiotic, a supplement, a fatty component, a milk product or a sweetening agent.

16. Food composition according to any one of the preceding claims, wherein the sweetening agent is a sugar, a high-intensity sweetener or a sugar substitute.

17. Food composition according to any one of the preceding claims, wherein the prebiotic is (are) inulin, oligofructose and/or galacto-oligosaccharides.

18. Food composition according to any one of the preceding claims, wherein the high-intensity sweetener is selected from the group consisting of sucralose, sodium cyclamate, acesulfame K, neohesperidin dihydrochalcone, glycyrrhizin, stevioside, monellin, thaumatin, aspartame, dulcin, saccharin, naringin dihydrochalcone, neotame, and a mixture of two or several thereof.

19. Food composition according to any one of the preceding claims, wherein the sugar substitute is selected from the group consisting of isomalt, 1,1-GPM (1-O-α-D-Glucopyranosyl-D-mannitol), 1,6-GPS (6-O-α-D-Glucopyranosyl-D-sorbit), 1,1-GPS (1-O-α-D-Glucopyranosyl-D-sorbitol), maltodextrins, lactitol, maltitol, erythritol, xylitol, mannitol, sorbitol, maltitol syrup, hydrogenated and non-hydrogenated starch hydrolysates and a mixture of two or several thereof.

20. Food composition according to any one of the preceding claims, wherein at least the one additive is a sugar, particularly saccharose, glucose, fructose, lactose, maltose or a mixture of two or several thereof.

21. Food composition according to any one of the preceding claims, wherein the at least one additive is a milk product, particularly a lactose-free milk product.

22. Food composition according to any one of the preceding claims, wherein the milk product is skim milk powder, whole milk powder, lactose-free skim milk powder, lactose-free whole milk powder, whey solid, or a mixture of two or several thereof.

23. Food composition according to any one of the preceding claims, wherein the at least one additive is a supplement selected from the group consisting of malt extract, flavoring agents, dyes, preserving agents, flavors, release agents, flavors, flow agents, mineral nutrients like sodium and calcium, particularly salts such as sodium chloride, vitamins, folic acid, emulsifiers, dietary fibers, lecithin, omega-3-fatty acids, medium chain length triglycerides, phythoestrogens and ascorbic acid salts.

24. Food composition according to any one of the preceding claims, wherein the probiotic is lactobacilli or bifidobacteria.

25. Food composition according to any one of the preceding claims, wherein isomaltulose or leucrose is the one and only body-giving sweetening agent that is present in the food composition.

26. Food composition according to any one of the preceding claims, wherein isomaltulose or leucrose is the one and only sugar that is present in the food composition.

27. Food composition according to any one of the preceding claims, wherein isomaltulose or leucrose is the one and only sweetening agent that is present in the food composition.

28. Use of isomaltulose and/or leucrose in a food, luxury food or pharmaceutical containing an emulsion with triglyceride oils to increase the stability of the emulsion in the food, luxury food or pharmaceutical.

## Revendications

1. Composition alimentaire de contrôle du poids et/ou de l'appétit, comprenant au moins deux ingrédients présentant chacun au moins une fonctionnalité ayant un effet positif en termes de physiologie nutritionnelle, le premier ingrédient présentant une fonctionnalité de contrôle de l'appétit et le second ingrédient une fonctionnalité à faible index glycémique, les deux ingrédients étant présents dans la composition alimentaire en une quantité apte à déployer l'effet positif en termes de physiologie nutritionnelle, le premier ingrédient étant une émulsion ayant une teneur en huiles triglycérides, les huiles triglycérides étant de l'huile de palme ou du beurre de cacao, et le second ingrédient étant de l'isomaltulose ou du leucrose, et l'émulsion étant une émulsion huile/eau à base d'huiles triglycérides combinées à un émulsifiant de qualité alimentaire dans une solution aqueuse, l'émulsifiant de qualité alimentaire étant un galactolipide ou de la lécithine.

2. Composition alimentaire selon la revendication 1, les huiles triglycérides étant de l'huile de palme fractionnée.

3. Composition alimentaire selon la revendication 1 ou 2, le galactolipide étant de l'huile d'avoine, tout particulièrement de l'huile d'avoine fractionnée.

4. Composition alimentaire selon l'une quelconque des revendications précédentes, la phase huileuse de l'émulsion contenant 80 à 99 % en poids d'huiles triglycérides et 1 à 20 % en poids d'émulsifiant de qualité alimentaire (respectivement par rapport à la phase huileuse).

5. Composition alimentaire selon l'une quelconque des revendications précédentes, les huiles triglycérides contenant au moins 90 % en poids de triglycérides.

6. Composition alimentaire selon l'une quelconque des revendications précédentes, les huiles triglycérides étant présentes avec de l'huile de noix de coco ou de l'huile de palmiste.

7. Composition alimentaire selon l'une quelconque des revendications précédentes, la teneur lipidique totale de l'émulsion étant de 40 à 50 % en poids (par rapport au poids total de l'émulsion).

8. Composition alimentaire selon l'une quelconque des revendications précédentes, la composition alimentaire contenant 1 à 50 % en poids (par rapport à la substance sèche totale de la composition alimentaire) du premier ingrédient.

9. Composition alimentaire selon l'une quelconque des revendications précédentes, la composition alimentaire contenant 1 à 50 % en poids du second ingrédient (par rapport à la substance sèche totale de la composition alimentaire).

10. Composition alimentaire selon la revendication 8, la composition alimentaire contenant 5 à 30 % en poids du premier ingrédient (par rapport à la substance sèche totale de la composition alimentaire).

11. Composition alimentaire selon la revendication 9, la composition alimentaire contenant 5 à 30 % en poids du second ingrédient (par rapport à la substance sèche totale de la composition alimentaire).

12. Composition alimentaire selon l'une quelconque des revendications précédentes, la composition alimentaire étant un aliment, une denrée ou un médicament solide, semi-solide ou liquide.

13. Composition alimentaire selon l'une quelconque des revendications précédentes, la composition alimentaire contenant 3 à 50 % en poids du premier ingrédient et 3 à 50 % en poids du second ingrédient (respectivement par rapport à la substance sèche totale de la composition alimentaire).

14. Composition alimentaire selon l'une quelconque des revendications précédentes, la composition alimentaire contenant au moins un additif.

15. Composition alimentaire selon l'une quelconque des revendications précédentes, le au moins un additif étant un prébiotique, un probiotique, un supplément, un composant gras, un produit laitier ou un agent édulcorant.

16. Composition alimentaire selon l'une quelconque des revendications précédentes, l'agent édulcorant étant un sucre, un édulcorant intensif ou un succédané de sucre.

17. Composition alimentaire selon l'une quelconque des revendications précédentes, le prébiotique étant de l'inuline, de l'oligofructose et/ou des galactooligosaccharides.

18. Composition alimentaire selon l'une quelconque des revendications précédentes, l'édulcorant intensif étant sélectionné dans le groupe consistant en sucralose, cyclamate de sodium, acésulfame K, dihydrochalcone de néohespéridine, glycyrrhizine, stévioside, monelline, thaumatine, aspartame, dulcine, saccharine, dihydrochalcone de naringine, néotame et un mélange de deux ou plus de ceux-ci.

19. Composition alimentaire selon l'une quelconque des revendications précédentes, le succédané de sucre étant sélectionné dans le groupe consistant en isomalte, 1,1-GPM (1-O-α-D-glucopyranosyl-D-mannite), 1,6-GPS (6-O-α-D-glucopyranosyl-D-sorbite), 1,1-GPS (1-O-α-D-glucopyranosyl-D-sorbite), maltodextrine, lactite, maltite, érythritol xylite, mannite, sorbite, sirop de maltite, hydrolysats d'amidon hydrogénés et non hydrogénés et un mélange de deux ou plus de ceux-ci.

20. Composition alimentaire selon l'une quelconque des revendications précédentes, le au moins un additif étant un sucre, tout particulièrement du saccharose, du glucose, du fructose, du lactose, du maltose ou un mélange de deux ou plus de ceux-ci.

21. Composition alimentaire selon l'une quelconque des revendications précédentes, le au moins un additif étant un produit laitier, tout particulièrement un produit laitier sans lactose.

22. Composition alimentaire selon l'une quelconque des revendications précédentes, le produit laitier étant de la poudre de lait écrémé, de la poudre de lait entier, de la poudre de lait écrémé sans lactose, de la poudre de lait entier sans lactose, un produit à base de lactosérum ou un mélange de deux ou plus de ceux-ci.

23. Composition alimentaire selon l'une quelconque des revendications précédentes, le au moins un additif étant un supplément sélectionné dans le groupe consistant en extrait de malte, aromatisants, colorants, conservateurs, arômes, antiagglomérants, agents aromatisants, agents fondants, substances minérales telles que sodium et calcium, tout particulièrement sels tels que chlorure de sodium, vitamines, acide folique, émulsifiants, fibres alimentaires, lécithine, acides gras oméga 3, triglycérides à chaîne moyenne, phytoestrogènes et sels d'acide ascorbique.

24. Composition alimentaire selon l'une quelconque des revendications précédentes, le probiotique étant des lactobacilles ou des bifidobactéries.

25. Composition alimentaire selon l'une quelconque des revendications précédentes, l'isomaltulose ou le leucrose étant le seul et unique agent édulcorant texturant présent dans la composition alimentaire.

26. Composition alimentaire selon l'une quelconque des revendications précédentes, isomaltulose ou le leucrose étant le seul et unique sucre présent dans la composition alimentaire.

27. Composition alimentaire selon l'une quelconque des revendications précédentes, isomaltulose ou le leucrose étant le seul et unique agent édulcorant présent dans la composition alimentaire.

28. Utilisation de isomaltulose ou du leucrose dans un aliment, une denrée de luxe ou un médicament contenant une émulsion à base d'huiles triglycérides, pour augmenter la stabilité de l'émulsion dans l'aliment, la denrée de luxe ou le médicament.
